# EUROPEAN PATENT SPECIFICATION

(11) **EP 1 366 347 B1**
(45) Date of publication and mention of the grant of the patent: **11.11.2009**
(21) Application number: 01917351.7
(22) Date of filing: 09.03.2001
(51) Int. Cl.: G01N 1/00

(54) **ULTRASENSITIVE NON-ISOTOPIC WATER-SOLUBLE NANOCRYSTALS**
HOCHEMPFINDLICHE NICHT-ISOTOPISCHE WASSERLÖSLISCHE NANOKRISTALLE
NANOCRISTAUX ULTRASENSIBLES, NON ISOTOPES, SOLUBLES DANS L'EAU

(43) Date of publication of application: 03.12.2003
(60) Divisional application: 09166316.1
(73) Proprietor: L'UNIVERSITE DE REIMS CHAMPAGNE-ARDENNE, 51097 Reims cedex (FR); SCIENTIFIC DEVELOPMENT SERVICE STROISNABSERVICE, 113035 Moscow (RU)
(72) Inventor: Nabiev, Igor, 51100 Reims (FR); Sukhanova, Alyona, 51100 Reims (FR); Jardillier, Jean-Claude, 51100 Reims (FR); Sharapov, Oleg, 31011 Kharkov (UA); Artemyev, Mikhail, Minsk (BY); Baranov, Alexandre, Saint Petersbourg (RU)
(74) Representative: Fosse, Danièle
(86) International application number: PCT/IB2001/000478
(87) International publication number: WO 2002/073155

(56) References cited:
- WO-A-00/17655
- WO-A-00/17656
- WO-A-00/27365
- WO-A-00/29617
- WO-A-00/58731
- CHAN WCW ET AL: "Quantum Dot bioconjugates for ultrasensitive nonisotopic detection" SCIENCE, AMERICAN ASSOCIATION FOR THE ADVANCEMENT OF SCIENCE,, US, vol. 281, 25 September 1998 (1998-09-25), pages 2016-2018, XP002125871 ISSN: 0036-8075

## Description

The present invention relates to ultrasensitive non-isotopic water-soluble nanocrystals for use in non-isotopic detection systems especially as probes for biological applications wherein the probes are able of providing a detectable electron paramagnetic resonance (EPR) signal or a fluorescence signal or combination thereof in response to exposure to electromagnetic radiation.

Non-isotopic detection systems have become a preferred mode rather than the use of radioactive markers in scientific research and clinical diagnostics for the detection of biomolecules using various assays such as DNA sequencing, nucleic acid amplification, immunohistochemistry, etc.

The semiconductor nanocrystals (quantum dots) are useful in biological applications as they can be rendered water-soluble, i.e. sufficiently soluble or suspendable in a aqueous-based solution such as in water or water-based solutions or buffer solutions including those used in biological or molecular detection systems.

To this aim, WO-A-0027365 proposes a composition of functionalized nanocrystals comprising quantum dots capped with a capping compound comprising mercaptocarboxylic acid forming a first layer and a second layer comprising diaminocarboxylic acid linked to the capping compound and further layers including amino acid, or affinity ligand linked to the diaminocarboxylic acid.

In WO-A-00/58731, the nanoparticles are made water-soluble by linking with a water-soluble amino derivative of a polysaccharide.

WO-A-00/28088 and WO-A-00/28089 describe water-soluble nanocrystals of the type described in WO-A-0027365 having polynucleotide strands to form dendrimers or nucleobases that can be detected by fluorescence emission.

WO-A-00/17656 describes a water-soluble semiconductor nanocrystal comprising as solubilization agent SH(CH₂)ₙX wherein X is carboxylate or sulfonate.

WO-A-00/17655 describes a water-soluble semiconductor nanocrystal comprising as solubilization agent a molecule having an hydrophobic region and an hydrophilic group consisting of carboxylic acid, carboxylate, sulfonate, hydroxide, alkoxides, ammonium salts, phosphate, phosphonate, methacrylic acid, acrylic acid, hydrophilically derivatized styrene, or molecules having the formula (R¹)ₐ-R²-(R³)_{b}(R⁴)_{c}]_{d} wherein R¹ is selected from the group consisting of heteroalkyl, heteroalkenyl, heteroalkynyl, -OR, -SR, -NHR, -NR'R'', -N(O)HR, -N(0)R'R'', -PHR, -PR'R'', - P(HR'R'')NR'R'', -P(O)(NR'R'')NR'R'', -P(O)(OR')OR'', - P(O)OR, -P(O)NR'R'', -P(S)(OR')OR'', and -P(S)OR wherein R, R' and R'' are independently selected from the group consisting of H, a branched or unbranched alkyl, a branched or unbranched alkenyl, a branched or unbranched heteroalkyl, a branched or unbranched heteroalkenyl and a branched or unbranched heteroalkynyl with the proviso that when a is greater than 1, the R' groups can be same or different or can be linked to form a six-, seven-, eight-, nine- or ten-membered cycloalkyl, cycloalkenyl, heterocyclic, aryl, heteroaryl or a six- to thirty membered crown ether or heterocrown ether ; R² is selected from a bond, a branched or unbranched alkylene, a branched or unbranched heteroalkylene, cycloalkyl, cycloalkenyl, heterocyclic aryl and heteroaryl, R³ is selected from a branched or unbranched alkylene, a branched or unbranched alkenylene, a branched or unbranched heteroalkylene, a branched or unbranched heteroalkenylene, cycloalkyl, cycloalkenyl, cycloalkynyl, heterocyclic aryl and heteroaryl, R⁴ is selected from the group consisting of an hydrogen, a carboxylate, a thiocarboxylate, an amine, an amide, an imine, an hydrazine, a sulfonate, a sulfoxide, a phosphate, a phosphonate, a phosphonium, an alcohol, a thiol, an ammonium, an alkyl ammonium, a nitrate, a sugar moiety and a five, six, seven, eight, nine, ten-membered cycloalkyl, cycloalkenyl, cycloalkynyl, heterocyclic aryl or heteroaryl, a being 1, 2, 3 or 4, b being 0, 1, 2 or 3, c being 0, 1, 2 or 3, d being 0, 1, 2 or 3. WO-A-00/17642 describes a composition comprising fluorescent semiconductor nanocrystals associated to a compound, the spectral emission of the nanocrystals providing information about a biological state or event, the nanocrystals being water-soluble because of a ligand having at least a linking group linked to the nanocrystal and an hydrophilic group.

WO-A-00/29617 describes water-soluble luminescent quantum dot and biomolecular conjugate thereof for ultrasensitive non-isotopic detection in vitro and in vivo. The water-soluble luminescent quantum dot comprises a core consisting of a nanoparticle-sized semiconductor (CdS or CdSe), a cap consisting of a semiconductor differing from the one of the core (ZnS or CdS) and an hydrophilic attachment group consisting of any organic group that can be attached to the surface of the cap and renders the quantum dot water-soluble. The binding between the cap and the hydrophilic attachment group is realised via a sulfur atom.

The water-soluble luminescent semiconductor quantum dot can then be linked directly or indirectly with a biomolecule like a protein, a fragment of protein or a nucleic acid, via the hydrophilic attachment group.

The aim of the present invention is to propose a water-soluble nanocrystal prepared with novel solubilisation agents and the method of water-solubilisation of the nanocrystals. Also provided are water-soluble semiconductor nanocrystals probes for biological application wherein the probes are able of providing a detectable electron paramagnetic resonance (EPR) signal or a fluorescence signal or a combination thereof in response to exposure to electromagnetic radiation.

The water-soluble semiconductor nanocrystal according the invention comprises semiconductor core and shell as well as a cap of one or more additional compounds comprising water-solubilization agents, and is **characterized in that** the water-solubilization agent is chosen in the group consisting of hydroxamates or derivatives of hydroxamic acid or a combination thereof.

The core is a semiconductor nanoparticle. While any core of the IIA-VIB, IIIA-VB or IVA-VIB semiconductors can be used, the core must be such that a luminescent quantum dot results upon combination with a shell. The core is preferably CdSe or ZnSe.

The shell is a semiconductor that differs from the one of the core and bonds to the core and is preferably a IIA-VIB semiconductor of a band gap higher than the core band gap, preferably ZnS, CdS or ZnSe.

According to one embodiment of the invention, the water-solubilizing agent is hydroxamate or a derivative of hydroxamic acid or a combination thereof.

Accordingly, nanocrystal powder is dissolved in an aliphatic solvent and then mixed with a water solution of derivative of hydroxamic acid, the aliphatic layer containing the nanocrystals is extracted and the nanocrystals are precipitated by addition of an alcohol solvent, the precipitate being then dried to powder or dissolved in water.

Preferably, the nanocrystal according the invention is doped with paramagnetic ions, such as Mn²+ and other transition or rare-earth ions.

The invention also provides a water-soluble nanocrystal probe comprising a semiconductor nanocrystal, a water-solubilisation agent, a linking agent and an affinity moiety which is able to bound specifically to detectable biological substances, **characterized in that** the water-soluble nanocrystal is according to claim 1.

According to one embodiment, the linking agent is able to connect the water-soluble nanocrystal or the polymeric bead comprising one or more nanocrystals embedded within with the affinity molecules including peptides, proteins, monoclonal or polyclonal antibodies and their functionnal fragments, oligonucleotides, RNA, single and double stranded DNA, RNA-DNA hymeric molecules, DNA triplexes and multiplexes and carbohydrates which are able to bound specifically to detectable biological substances, e.g. proteins, oligonucleotides, DNA, RNA, viruses, bacteria, cells, including living cells and tissues.

Preferably, the water-solubilisation agent is also the linking agent.

The nanocrystals probes consisting of polymeric beads should have the same field of application as widely used known Nanofluorospheres comprising organic dyes molecules embedded in the polymeric beads. Advantages of the polymeric beads with the embedded nanocrystals doped or undoped with the paramagnetic ions, compared with the Nanofluorospheres are as follows :
- 100-fold improved stability of nanocrystals against photo-bleaching as compared to organic dyes ;
- possibility of multicolour labelling using the nanocrystals of different diameters and of different core and shell ;
- possibility of using probes comprising nanocrystals doped with the paramagnetic ions as fluroescence or magnetic resonance probes or as a combination thereof.

The invention further comprises the method of detection of biological substances by registration of EPR signal from paramagnetic ions incorporated into the nanocrystals or of fluorescence signal from the nanocrystals or a combination thereof or by the construction of the images of biological substances by fluorescence or EPR microscopy or by a combination thereof.

The biological imaging is provided by scanning the object by following ways :
- via detection of spacial distribution of the nanocrystal flurorescence by fluorescence microscopy ,
- via detection of the EPR signal with high spatial resolution by technique analogous to the scanning tunnel microscopy with a ferromagnetic crystal needle producing the local magnetic field (Hochi A, Furusawa M, Ikeya M, Applications of microwave scanning ESR microscope : human tooth with metal. Appl Radiat Isot 1993 Jan-Feb;44(1-2):401-405) or by cavity technique with the use of a narrow aperture for penetration of the microwave electromagnetic field in the object;
- via simultaneous detection of the EPR signal with high spatial resolution and of the fluorescence signal with high spatial resolution.

The invention will be further described in more details in reference to the following examples and to the annexed drawing wherein :
Figure 1 is a schematic illustration of the doped nanocrystals of the invention ;
Figure 2 is a schematic illustration of the water-solubilisation of the nanocrystals with hydroxamates of amino acids or with compounds possessing hydroxamic acid functional group.
Figure 3 is a schematic illustration of anchoring of the affinity molecules to the water-soluble nanocrystal capped with the hydroxamate of amino acid or with compounds possessing hydroxamic acid functional group.

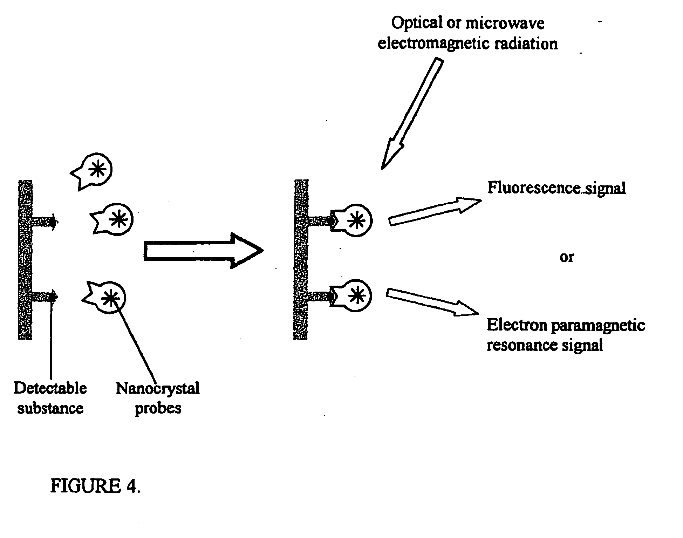

Figure 4 is a schematic representation of the use of water-soluble nanocrystal probe of the invention linked with the affinity molecule for ultrasensitive non-isotopic detection and analysis of a detectable substance in a biological material by fluorescence or by electron paramagnetic resonance (EPR) technique or by the combination thereof.
Figures 5a, 5b, 5c are examples of applications of the water-soluble nanocrystals probes according the invention for detection of biological objects.

The following examples serve to illustrate the present invention and are not intended to limit the scope of the invention.

### Example 2 : water-solubilisation of nanocrystal capped with the glycine hydroxamate and the binding thereof to affinity biological molecules.

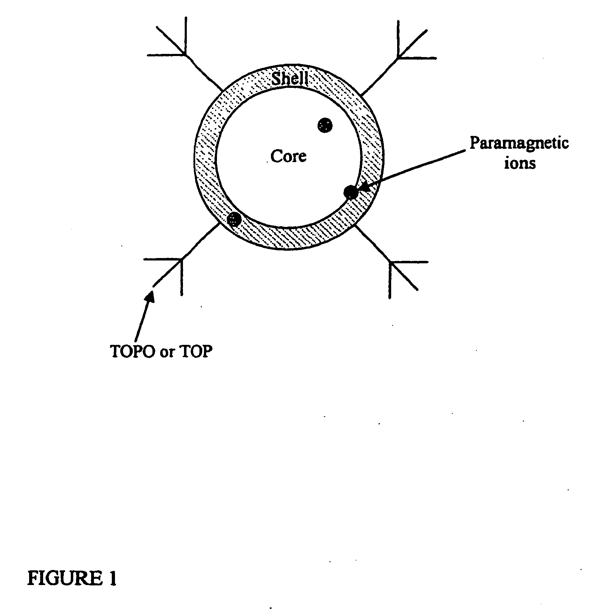

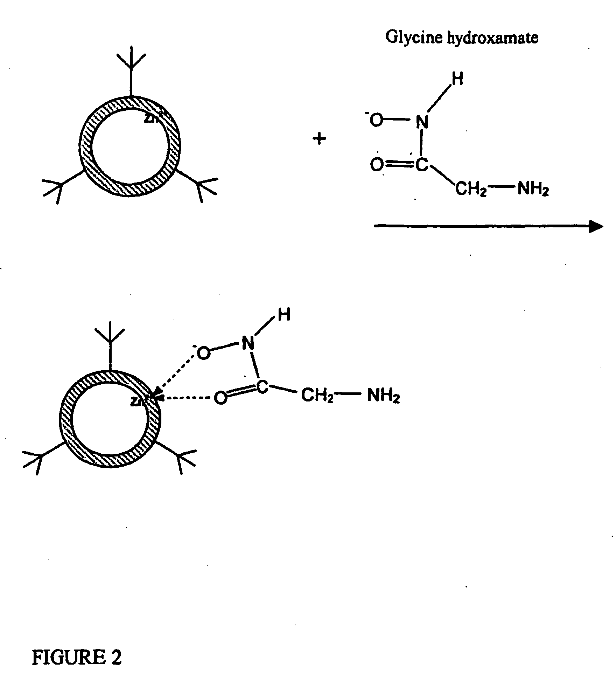

Few milligrams of nanocrystals powder are dissolved in heptane and mixed with water solution of glycine hydroxamate (0,1-0,001 M) under vigorous stirring. Approximately in one hour, the nanocrystals are extracted from the aliphatic layer due to bonding of the hydroxamate to the Zn-atoms on the nanocrystal surface. The aliphatic layer is further removed and nanocrystals are precipated by adding methanol, ethanol, propanol or acetone. The precipitate is dried to powder, or dissolved in fresh portion of water for further manipulations (figures 1 and 2).

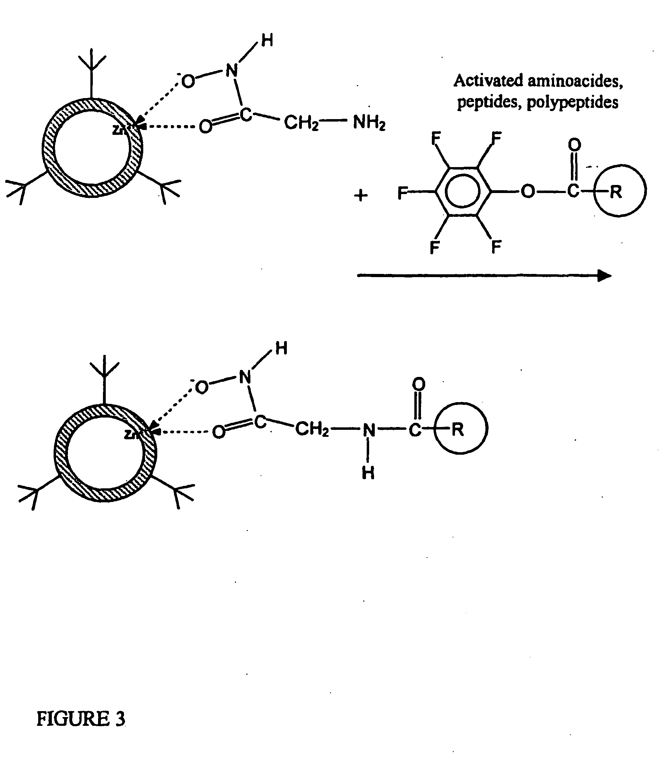

The binding of such water-soluble nanocrystals with biomolecules of interest such as amino acids, peptides, proteins, monoclonal or polyclonal antibodies and their functional fragments, oligonucleotides, RNA, single or double-stranded DNA, RNA-DNA hymeric molecules, DNA triplexes and multiplexes and carbohydrates, eg sialic acids, etc. may be further proceeded as described in Bioconjugate techniques, 1996, Ed.Hermanson, Academic Press, using the free NH₂-group of the nanocrystal-attached hydroxamate as an active linker group as presented in figure 3 wherein the nanocrystal probe reacts with activated aminoacids, peptides or polypeptides and R is amino acids, peptides, proteins, monoclonal or polyclonal antibodies and their functional fragments, oligonucleotides, RNA, single or double-stranded DNA, RNA-DNA hymeric molecules, DNA triplexes and multiplexes and carbohydrates, eg sialic acids, etc.

### Example 6 : use of the water-soluble doped or undoped nanocrystal probes for detection of biological substances and preparation of samples containing nanocrystal probes of the invention and recording of fluorescence image o biological object (living cell or in vitro immunological assays).

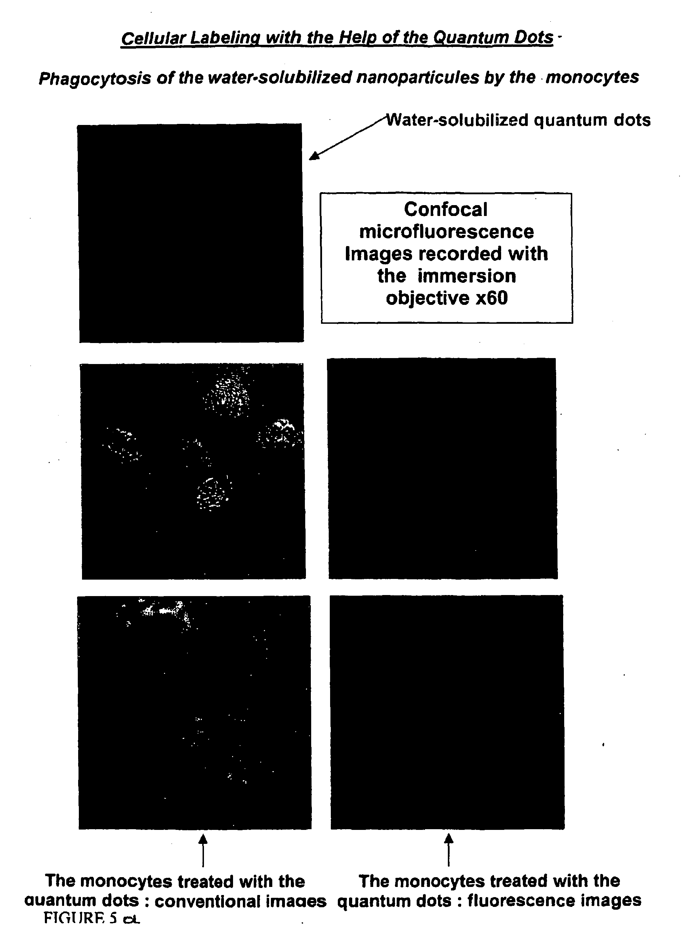

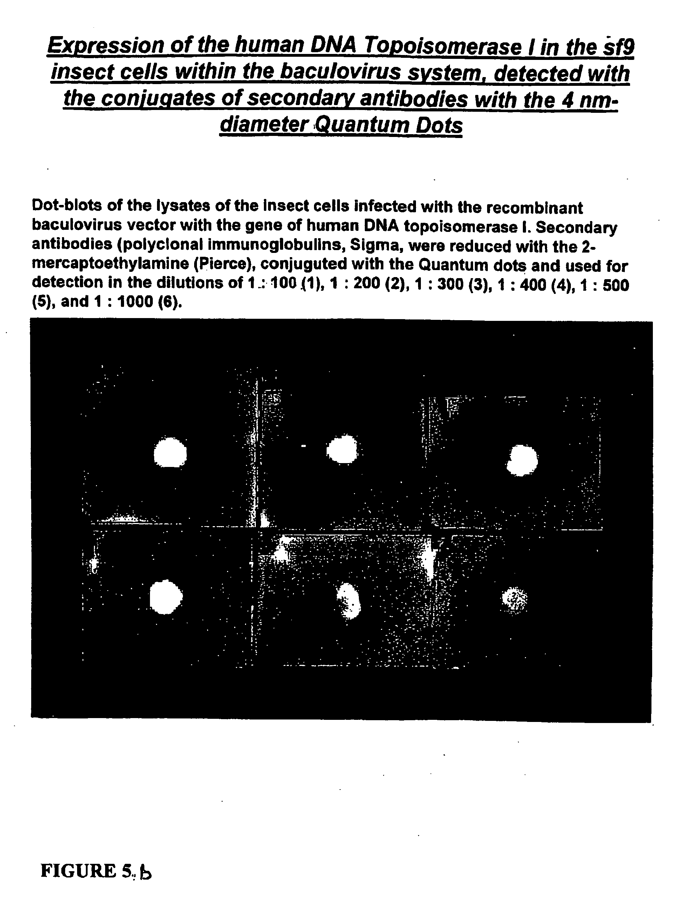

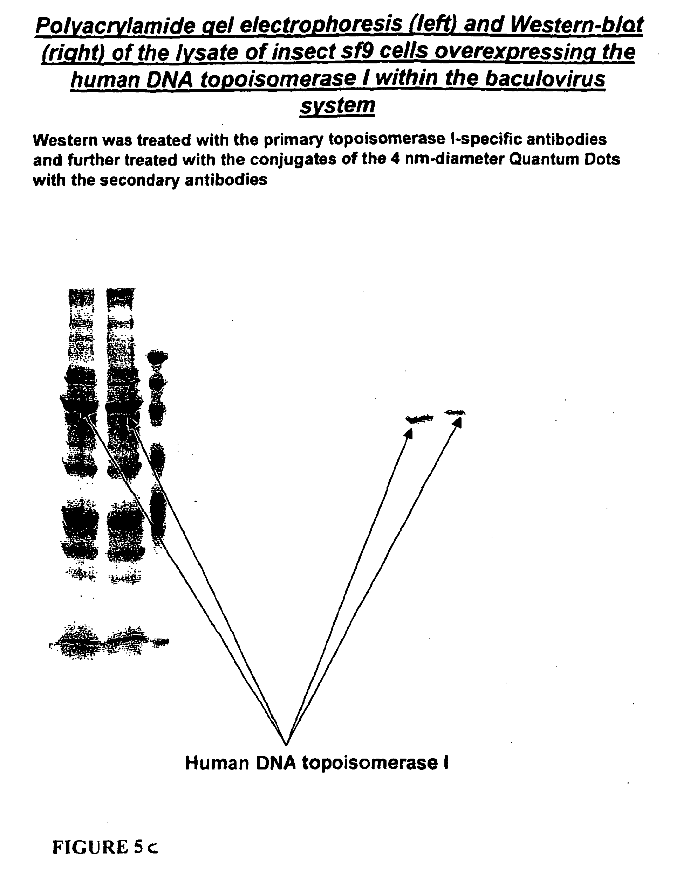

The figures 5a, 5b and 5c show examples of application of the water-soluble nanocrystals of the invention for fluorescence ex-vivo imaging of biological object (monocytes treated with the water-solubilized nanocrystals) and the results of application of the antibodies-nanocrystals conjugates in the Western blot and dot-blot immunoassays for ultrasensitive detection of biological substances

The samples were prepared using conventional techniques and the experimental conditions are presented in the figures.

The cellular labelling with the help of the quantum dots according to the invention is illustrated in figure 5a.

As seen in figure 5c, antibodies-nanocrystals conjugates are used in the Western blot. The Western blot was treated with the primary topoisomerase I-specific antibodies and further treated with the conjugates of the 4nm-diameter quantum dots with the secondary antibodies.

As seen in figure 5b, antibodies-nanocrystals conjugates are used in dot-blot immunoassays for ultrasensitive detection of biological substances.

## Claims

1. Water-soluble semiconductor nanocrystal comprising semiconductor core and shell as well as a cap of one or more additional compounds comprising water-solubilization agents, **characterized in that** the water-solubilization agent is chosen in the group consisting of hydroxamates or derivatives of hydroxamic acid or a combination thereof.

2. Water-soluble semiconductor nanocrystal according to claim 1,
**characterised in that** the nanocrystal is doped with paramagnetic ions.

3. Water-soluble nanocrystal probe comprising a water soluble semiconductor nanocrystal according to claim 1, a water-solubilisation agent, a linking agent and an affinity moiety which is able to bound specifically to detectable biological substances.

4. Probe according to claim 3,
**characterized in that** the linking agent is able to connect the water-soluble nanocrystal or the polymeric bead comprising one or more nanocrystales embedded within, with the affinity molecules including peptides, proteins, monoclonal or polyclonal antibodies and their functionnal fragments, oligonucleotides, RNA, single and double stranded DNA, RNA-DNA hymeric molecules, DNA triplexes and multiplexes and carbohydrates which are able to bound specifically to detectable biological substances.

5. Probe according to the claims 3 and 4,
**characterized in that** the water-solubilisation agent is also the linking agent.

6. Process for the preparation of a water-soluble semi-conductor nanocrystal according to claim 1,
**characterised in that** a nanocrystal powder is dissolved in an aliphatic solvent and then mixed with a water solution of derivative of hydroxamic acid, the aliphatic layer containing the nanocrystals is extracted and, the nanocrystals are precipitated by addition of an alcohol solvent, the precipitate being then dried to powder or dissolved in water.

7. Method of detection of biological substances by registration of EPR signal from paramagnetic ions incorporated into the nanocrystals according to claim 2.

8. Method of detection of biological substances by registration of fluorescence signal from the nanocrystal according to claims 1 or 2.

9. Method of detection of biological substances by registration of a combination of fluorescence signal and EPR signal from paramagnetic ions incorporated into the nanocrystals according to claim 2.

10. Method of detection of biological substances by the construction of images of biological substances by fluorescence or EPR microscopy or by a combination thereof from the nanocrystals according to claims 1 or 2.

## Patentansprüche

1. Wasserlöslicher Halbleiternanokristall mit Halbleiterkern und Halbleiterschale sowie einer Schicht aus einem oder mehreren zusätzlichen Verbindungen mit Lösungsvermittlern, **dadurch gekennzeichnet, dass** das Wasser-Solubilisierungsmittel aus der Gruppe bestehend aus Hydroxamaten oder Derivaten der Hydroxamsäure oder einer Kombination davon gewählt wird.

2. Wasserlöslicher Halbleiternanokristall nach Anspruch 1, **dadurch gekennzeichnet, dass** der Nanokristall mit paramagnetischen Ionen dotiert ist.

3. Wasserlösliche Nanokristall-Probe mit einem wasserlöslichen Halbleiternanokristall nach Anspruch 1, einem Wasser-Solubilisierungsmittel, einem Verbindungsmittel und einer Affinitätskomponente, die sich spezifisch an nachweisbare biologische Substanzen bindet.

4. Probe nach Anspruch 3, **dadurch gekennzeichnet, dass** das Verbindungsmittel den wasserlöslichen Nanokristall oder das darin eingebettete, einen oder mehrere Nanokristalle umfassende Polymerkügelchen mit den Affinitätsmolekülen einschließlich Peptiden, Proteinen, monoklonalen oder polyklonalen Antikörper und deren funktionellen Fragmente, Oligonucleotide, RNS, einzel- und doppelsträngige DNS, hymerische RNS/DNS-Moleküle, DNS-Triplex- und -Multiplex-Strukturen und Kohlenhydrate, die sich spezifisch an nachweisbare biologische Substanzen binden, verbindet.

5. Probe nach den Ansprüchen 3 und 4, **dadurch gekennzeichnet, dass** das Wasser-Solubilisierungsmittel auch das Verbindungsmittel ist.

6. Verfahren zur Herstellung eines wasserlöslichen Halbleiternanokristalls nach Anspruch 1, **dadurch gekennzeichnet, dass** ein Nanokristallpulver in einem aliphatischen Lösungsmittel gelöst wird und dann mit einer wässrigen Lösung eines Hydroxamsäurederivates gemischt wird, die die Nanokristalle enthaltende aliphatische Schicht extrahiert wird und die Nanokristalle durch Zugabe eines Alkohol-Lösungsmittels ausgefällt werden, wobei der Niederschlag dann zu Pulver getrocknet oder in Wasser gelöst wird.

7. Verfahren zum Nachweis biologischer Substanzen durch Aufzeichnung eines ESR-Signals von in den Nanokristallen nach Anspruch 2 enthaltenen paramagnetischen Ionen.

8. Verfahren zum Nachweis biologischer Substanzen durch Aufzeichnung eines Fluoreszenzsignals vom Nanokristall nach Anspruch 1 oder 2.

9. Verfahren zum Nachweis biologischer Substanzen durch Aufzeichnung einer Kombination aus einem Fluoreszenzsignal und einem ESR-Signal von in den Nanokristallen nach Anspruch 2 enthaltenen paramagnetischen Ionen.

10. Verfahren zum Nachweis biologischer Substanzen durch die Erstellung von Bildern von biologischen Substanzen durch Fluoreszenz- oder ESR-Mikroskopie oder durch eine Kombination davon von den Nanokristallen nach Anspruch 1 oder 2.

## Revendications

1. Nanocristal semi-conducteur soluble dans l'eau comprenant un noyau et une enveloppe semi-conducteurs ainsi qu'une capsule d'un ou plusieurs composés supplémentaires comprenant des agents de solubilisation dans l'eau, **caractérisé en ce que** l'agent de solubilisation dans l'eau est choisi dans le groupe constitué par les hydroxamates ou dérivés de l'acide hydroxamique ou leurs combinaisons.

2. Nanocristal semi-conducteur soluble dans l'eau selon la revendication 1,
**caractérisé en ce que** le nanocristal est dopé avec des ions paramagnétiques.

3. Sonde de nanocristaux solubles dans l'eau comprenant un nanocristal semi-conducteur soluble dans l'eau selon la revendication 1, un agent des solubilisations dans l'eau, un agent de liaison et un fragment d'affinité qui est capable de se lier spécifiquement à des substances biologiques détectables.

4. Sonde selon la revendication 3,
**caractérisée en ce que** l'agent de liaison est capable de relier le nanocristal soluble dans l'eau ou la bille polymère dans laquelle un ou plusieurs nanocristaux sont intégrés, aux molécules d'affinité comprenant des peptides, des protéines, des anticorps monoclonaux et polyclonaux et leur fragments fonctionnels, des oligonucléotides, de l'ARN, de l'ADN simple et double brin, des molécules d'hymères ARN-ADN, des triplex et multiplex d'ADN et des glucides qui sont capables de se lier spécifiquement à des substances biologiques détectables.

5. Sonde selon les revendications 3 et 4,
**caractérisée en ce que** l'agent de solubilisation dans l'eau est également l'agent de liaison.

6. Processus pour la préparation d'un nanocristal semi-conducteur soluble dans l'eau selon la revendication 1,
**caractérisé en ce qu'**une poudre de nanocristaux est dissoute dans un solvant aliphatique, puis mélangée à une solution aqueuse d'un dérivé de l'acide hydroxamique, la couche aliphatique contenant les nanocristaux est extraite et, les nanocristaux sont précipités par ajout d'un solvant alcoolique, le précipité étant alors séché pour être réduit en poudre ou dissous dans l'eau.

7. Procédé de détection de substances biologiques par enregistrement d'un signal EPR à partir d'ions paramagnétiques incorporés dans les nanocristaux selon la revendication 2.

8. Procédé de détection de substances biologiques par enregistrement d'un signal de fluorescence provenant du nanocristal selon la revendication 1 ou 2.

9. Procédé de détection de substances biologiques par enregistrement d'une combinaison d'un signal de fluorescence et d'un signal EPR à partir d'ions paramagnétiques incorporés dans les nanocristaux selon la revendication 2.

10. Procédé de détection de substances biologiques par construction d'images des substances biologiques par fluorescence ou microscopie EPR ou par une combinaison de celles-ci, des nanocristaux selon la revendication 1 ou 2.
